# EUROPÄISCHE PATENTSCHRIFT

(11) **EP 1 121 460 B1**
(45) Veröffentlichungstag und Bekanntmachung des Hinweises auf die Patenterteilung: **14.07.2010**
(21) Anmeldenummer: 99923428.9
(22) Anmeldetag: 20.04.1999
(51) Int. Cl.: C12Q 1/68, C07H 1/08

(54) **VERFAHREN UND MITTEL ZUR ISOLIERUNG UND REINIGUNG VON NUKLEINSÄUREN AN OBERFLÄCHEN**
METHODS AND MEANS FOR ISOLATING AND PURIFYING NUCLEIC ACIDS ON SURFACES
PROCEDES ET MOYENS POUR L'ISOLEMENT ET LA PURIFICATION D'ACIDES NUCLEIQUES SUR DES SURFACES

(30) Priorität: 23.10.1998 WO PCT/EP98/06756
(43) Veröffentlichungstag der Anmeldung: 08.08.2001
(73) Patentinhaber: Qiagen Gesellschaft mit Beschränkter Haftung, 40724 Hilden (DE)
(72) Erfinder: WEBER, Martin, D-42799 Leichlingen (DE); FUHRMANN, Guido, D-41812 Erkelenz (DE); SCHORR, Joachim, D-40724 Hilden (DE)
(74) Vertreter: Zimmermann & Partner
(86) Internationale Anmeldenummer: PCT/EP1999/002664
(87) Internationale Veröffentlichungsnummer: WO 2000/024927

(56) Entgegenhaltungen:
- EP-A- 0 295 069
- EP-A- 0 389 063
- EP-A- 0 487 028
- EP-A- 0 512 768
- EP-A- 0 651 249
- WO-A-87/06621
- WO-A-87/07645
- WO-A-98/42874
- WO-A-99/22021
- US-A- 4 833 239
- US-A- 5 438 128
- RUPPERT A ET AL: "A filtration method for plasmid isolation using microtiter filter plates." ANALYTICAL BIOCHEMISTRY, (1995 SEP 1) 230 (1) 130-4., XP000611257 US

## Beschreibung

Die vorliegende Erfindung betrifft ein Verfahren zur Fällung von Plasmiden und die Verwendung von Membranen zur Bindung von Alkohol-gefällten Plasmiden.

Es ist seit langem bekannt, daß die genetische Herkunft und funktionelle Aktivität einer Zelle durch Studien ihrer Nukleinsäuren bestimmt und untersucht werden kann. Die Analysen der Nukleinsäuren ermöglichen den direkten Zugriff auf die Ursache der Aktivitäten von Zellen. Sie sind somit indirekten, konventionellen Methoden, wie z.B. dem Nachweis von Stoffwechselprodukten, potentiell überlegen. Daher ist für die Zukunft mit einer starken Verbreitung von Nukleinsäureanalysen zu rechnen. So werden molekularbiologische Analysen bereits in vielen Bereichen eingesetzt, z.B. in der medizinischen und klinischen Diagnostik, in der Pharmazie bei der Entwicklung und Evaluierung von Arzneimitteln, in der Lebensmittelanalytik sowie bei der Überwachung der Lebensmittelherstellung und der Lebensmittelkontrolle, in der Agrarwirtschaft bei der Züchtung von Nutzpflanzen und Nutztieren sowie in der Umweltanalytik und in vielen Forschungsgebieten. Hierzu zählen zum Beispiel die Vaterschaftsanalyse, Gewebetypisierungen, Identifizierung von Erbkrankheiten, Genomanalyse, molekulare Diagnostik wie z.B. bei der Identifizierung von Infektionskrankheiten, transgene Forschung, Grundlagenforschung auf dem Gebiet der Biologie und der Medizin sowie zahlreiche verwandte Arbeitsgebiete.

Durch die Analyse der RNA, speziell der mRNA in Zellen, lassen sich die Aktivitäten von Genen direkt bestimmen. Die quantitative Analyse von Transkriptmustern (mRNA-Mustern) in Zellen durch moderne molekularbiologische Methoden, wie z.B. Echtzeit-Reverse Transkriptase PCR ("Real-Time RT-PCR") oder Genexpressions-Chip-Analysen ermöglicht z.B. die Erkennung fehlerhaft exprimierter Gene, wodurch z.B. Stoffwechselkrankheiten, Infektionen oder die Entstehung von Krebs erkannt werden können. Die Analyse der DNA aus Zellen durch molekularbiologische Methoden, wie z.B. PCR, RFLP, AFLP oder Sequenzierung ermöglicht z.B. den Nachweis genetischer Defekte oder die Bestimmung des HLA-Typs sowie anderer genetischer Marker.

Die Analyse genomischer DNA und RNA wird auch zum direkten Nachweis von infektiösen Erregern, wie Viren, Bakterien usw. eingesetzt.

Dabei besteht eine generelle Schwierigkeit darin, biologische bzw. klinische Probenmaterialien so aufzubereiten, daß die in ihnen enthaltenen Nukleinsäuren direkt in die jeweilige Analysenmethode eingesetzt werden können. Gerade die direkte Verwendbarkeit bei guter Ausbeute und hoher Qualität der Nukleinsäuren; bei gleichzeitig hoher Reproduzierbarkeit, ist allerdings bei höheren Probenzahlen wichtig, wenn die Analysen automatisch ablaufen sollen.

Aus dem Stand der Technik sind bereits zahlreiche Verfahren zur Reinigung von DNA bekannt. So ist es bekannt, Plasmid-DNA beispielsweise zum Zwecke des Klonierens oder auch für andere experimentelle Vorhaben nach dem Verfahren von Birnboim [Methods in Enzymology 100 (1983), S. 243] zu reinigen. Nach diesem Verfahren wird ein geklärtes Lysat bakteriellen Ursprungs einem Caesiumchlorid Gradienten ausgesetzt und über einen Zeitraum von 4 bis 24 Stunden zentrifugiert. Diesem Verfahrensschritt folgt gewöhnlicherweise die Extraktion und die Präzipitation der DNA. Dieses Verfahren ist mit dem Nachteil verbunden, daß es zum einen apparativ sehr aufwendig und zum anderen sehr zeit- und kostenintensiv sowie nicht automatisierbar ist.

Andere Methoden, bei denen geklärte Lysate eingesetzt werden, um DNA zu isolieren, sind die Ionenaustauschchromatographie [Colpan et al., J. Chromatog. 296 (1984), S. 339] und die Gelfiltration [Moreau et al. Analyt. Biochem. 166 (1987), S. 188]. Diese Verfahren bieten sich in erster Linie als Ersatz für den Caesiumchlorid-Gradienten an, machen aber ein aufwendiges System für die Lösungsmittelversorgung sowie die Präzipitation der so gewonnenen DNA-Fraktionen erforderlich, da sie gewöhnlicherweise Salze in hoher Konzentration enthalten und sehr verdünnte Lösungen darstellen.

Marko et al. [Analyt. Biochem. 121 (1982), S. 382] sowie Vogelstein et al. [Proc. Nat. Acad. Sci. 76 (1979), S. 615] erkannten, daß, falls die DNA aus Nukleinsäure-enthaltenden Extrakten hohen Konzentrationen von Natriumiodid oder Natriumperchlorat ausgesetzt wird, nur die DNA an mechanisch fein zerkleinerten Glas-Scintillationsröhrchen sowie zerkleinerten Glasfasermembranen bzw. Glasfiberplatten bindet, während RNA und Proteine nicht binden. Die so gebundene DNA kann ggfs. mit Wasser eluiert werden.

So wird in der WO 87/06621 die Immobilisierung von Nukleinsäuren an einer PVDF-Membran beschrieben. Allerdings werden die an die PVDF-Membran gebundenen Nukleinsäuren anschließend nicht eluiert, sondern die Membran wird samt gebundener Nukleinsäuren direkt in einen PCR-Ansatz eingebracht. Letztendlich wird in dieser internationalen Patentanmeldung und in der weiteren Literatur jedoch die Lehre offenbart, daß hydrophobe Oberflächen bzw. Membranen im allgemeinen zuvor mit Wasser oder Alkohol benetzt werden müssen, um die Nukleinsäuren in halbwegs befriedigenden Ausbeuten immobilisieren zu können.

Für eine Reihe von modernen Applikationen, wie z. B. der PCR-, der Reversed-Transcription-PCR, SunRise, LCR, branched-DNA, NASBA, TaqMan-Technologie und ähnlicher Echtzeitquantifizierungsverfahren für PCR, SDA, DNA- und RNA-Chips und -Arrays zur Genexpressions- und Mutationsanalytik, differential display Analytik, RFLP, AFLP, cDNA-Synthesen, oder subtraktive Hybridisierung, ist es auf der anderen Seite jedoch absolut notwendig, die Nukleinsäuren direkt von der festen Phase lösen zu können. Hierzu ist der WO 87/06621 die Lehre zu entnehmen, daß die Nukleinsäure zwar von den dort eingesetzten Membranen wiedergewonnen werden kann, daß diese Wiedergewinnung jedoch sehr problematisch ist und bei weitem nicht zur quantitativen Isolierung von Nukleinsäuren geeignet ist. Daneben fallen die so gewonnenen Nukleinsäuren in vergleichsweise sehr hoher Verdünnung an - ein Umstand, der weitere Folgeschritte zwecks Konzentrierung und Isolierung zwangsläufig erforderlich macht.

Aus der EP-A-0 487 028 ist eine Vorrichtung zur Extrahierung und Reinigung von Nukleinsäuren bekannt. Die Vorrichtung umfasst eine Reihe von Gefäßen, welche mindestens eine Lösung zur Extrahierung und Reinigung von Nukleinsäuren enthalten, eine Einrichtung zum Ansaugen und Abgeben der Lösung, wobei die Einrichtung so angeordnet ist, dass sie entlang der Gefäße verschiebbar ist, sowie weitere Mittel zum Anbringen einer Filtereinheit an der Einrichtung zum Ansaugen und Abgeben der Lösung.

Die US-A-4,833,239 befasst sich mit einem Verfahren zur Isolierung und Reinigung von Nukleinsäuren aus prokaryotischen und eukaryotischen oder viralen Zellkulturen.

Die EP-A-0 512 768 beschreibt ein Verfahren zur Reinigung von DNA aus irgendeiner Quelle und in irgendeiner Form. Das Verfahren umfasst bei der Reinigung der DNA die Verwendung von wasserlöslichen organischen Lösungsmitteln. Durch die Verwendung wasserlöslicher organischer Lösungsmittel, wie Ethanol, Propanol oder Isopropanol wird die DNA mit größeren Ausbeuten gereinigt. Zudem wird durch die Verwendung wasserlöslicher organischer Lösungsmittel vermieden, dass ätzende und giftige Verbindungen, wie chaotrope Substanzen, verwendet werden müssen.

Die EP-A-0 295 069 betrifft eine diagnostische Testvorrichtung zur Ermittlung der Anwesenheit einer Verbindung in einer flüssigen Probe. Die Vorrichtung enthält ein flüssigkeitsundurchlässiges Behältnis, welches durch ein flüssigkeitsabstoßendes Element belüftet wird. Das Behältnis beinhaltet ein absorbierendes Mittel, welches mit einer Flüssigkeit in Kontakt steht und Flüssigkeit durch ein mikroporöses Medium zieht, wobei Gas während der Flüssigkeitsadsorption aus dem absorbierenden Mittel verdrängt wird. Das flüssigkeitsabstoßende Element belüftet das verdrängte Gas während es sicherstellt, dass die Flüssigkeiten innerhalb des Behältnisses verbleiben. Die diagnostische Testvorrichtung kann auch einen Deckel mit Öffnung aufweisen, die mit dem mikroporösen Medium kommuniziert, um Flüssigkeiten darauf aufzubringen, während er eine flüssigkeitsundurchlässige Abdichtung mit einer Wand des Behältnisses bildet. Der Deckel kann auch das mikroporöse Medium in positiven Kontakt mit dem absorbierenden Mittel zwingen, um die Absorption zu unterstützen.

Die WO-A-9842874 betrifft ein Verfahren zur Isolierung und Reinigung von Nukleinsäuren aus Blut, Plasma, Kulturen durch automatisierte Mittel ohne den Einsatz von Zentrifugation, Aspiration oder Vakuum.

Die US-A-5438128 beschreibt eine Vorrichtung, ein Verfahren und einen Kit zur schnellen Isolierung und Reinigung von Nukleinsäuren, Proteinen, Peptiden, Kohlenwasserstoffen und Oligosacchariden aus heterogenen biologischen Proben.

Unter Nukleinsäuren fallen im Sinne der vorliegenden Erfindung Plasmide.

Aus den oben genannten Gründen stellen die aus dem Stand der Technik bekannten Verfahren - insbesondere im Hinblick auf eine Automatisierung des Verfahrensablaufs zur Nukleinsäuregewinnung - keinen geeigneten Ausgangspunkt für eine verfahrenstechnisch möglichst einfache und quantitative Isolierung von Nukleinsäuren dar.

Es ist daher die Aufgabe der vorliegenden Erfindung, die Nachteile der aus dem Stand der Technik bekannten Verfahren zur Isolierung von Nukleinsäuren zu überwinden und Verfahren und Mittel zur Verfügung zu stellen, welche dazu geeignet sind, ohne erheblichen technischen Aufwand durchgeführt bzw. verwendet werden zu können.

Gelöst wird die Aufgabe erfindungsgemäß durch das Verfahren gemäß des unabhängigen Patentanspruchs 1 sowie der Verwendung gemäß und des unabhängigen Anspruchs 5.

Weitere vorteilhafte Aspekte und Ausgestaltungen der Erfindung ergeben sich aus den abhängigen Patentansprüchen.

Dabei ist die Erfindung auf ein Verfahren gerichtet, das poröse Membranen aus Celluloseacetat oder Cellulosenitrat verwendet, an welcher die Nukleinsäuren auf einfache Weise aus der die Nukleinsäuren enthaltenden Probe immobilisiert und mittels ebenso einfacher Verfahrensschritte wieder abgelöst werden können, wobei es die erfindungsgemäß einfache Prozeßführung ermöglicht, die Verfahren insbesondere vollautomatisch durchführen zu können.

Ein weiterer Aspekt der vorliegenden Erfindung ist darauf gerichet, Nukleinsäuren an eine an eine Membran in der Art und Weise zu binden, daß sie in einem folgenden Reaktionsschritt ohne weiteres wieder von dieser Phase abgelöst werden können und ggf. in weiteren Anwendungen - wie z. B. Restriktionsverdauung, RT, PCR oder RT-PCR oder in jedweder anderen oben genannten geeigneten Analyse- bzw. Enzymreaktion eingesetzt werden können.

Von einer Membran im Sinne der vorliegenden Erfindung ist dann die Rede, wenn es sich um eine Trennschicht handelt, die von beiden Seiten zugänglich ist, also nicht mit ihrer gesamten Fläche auf einem undurchlässigen Untergrund aufliegt, sondern ganz frei oder nur an einzelnen Punkten unterstützt ist.

Unter Isolierung wird im Sinne der vorliegenden Erfindung dabei jede Anreicherung von Nukleinsäuren verstanden, bei der also die Konzentration der Nukleinsäuren erhöht und/oder der Anteil an Nichtnukleinsäure in einem Ansatz bzw. einer Probe verringert wird.

Bei vorbekannten, auf Anionenaustauscherchromatographie basierenden Methoden zur Plasmid-DNA Aufreinigung von 100 µg und mehr (im folgenden als "Großmaßstab" bezeichnet) wird, im letzten Schritt, die Plasmid DNA in einem Hochsalzpuffer von der Säule eluiert. Um die Plasmid DNA zum einen vom Salz zu trennen und sie zum anderen zu konzentrieren, wird sie mit Hilfe von Alkoholen (z.B. Isopropanol) präzipitiert und in einem geeigneten Gefäß abzentrifugiert. Das erhaltene Zentrifugationspellet wird mit 70%igem Ethanol gewaschen, um restliche Spuren von Salz zu entfernen, und dann nochmals einer Zentrifugation unterzogen. Das Pellet dieser zweiten Zentrifugation wird typischerweise in einer geringen Menge Niedrigsalzpuffer gelöst und die Plasmid-DNA in dieser Form weiterverwendet.

In Stand der Technik sind zudem Verfahren vorgeschlagen worden, die DNA durch Zugabe von chaotropen Salzen in den Hochsalzpuffer in eine Form zu überführen, die an Silikamembranen bindet. Nach einem entsprechenden Waschschritt kann die DNA durch einen Niedrigsalzpuffer wieder von der Membran gelöst werden.

In einer Veröffentlichung (Ruppert, A. et al. , Analytical Biochemistry (1995), 230: 130-134) wird eine ähnliche Anwendung beschrieben, bei der im Kleinmaßstab (Isolierung von weniger als 100 µg Plasmid-DNA) eine Isopropanol gefällte DNA an PVDF-Membranen einer Porengröße von kleiner als 0,2 µm bindet, daraufhin mit Ethanol gewaschen und anschließend mit TE (Tris-EDTA) eluiert wird. Es existiert jedoch keine Beschreibung einer derartigen Methode im Großmaßstab.

Die beschriebene Fällung von DNA mit anschließender Zentrifugation ist extrem zeitaufwendig (etwa 1 Stunde), zudem ist der Einsatz von Zentrifugen notwendig. Neben dem hohen Zeitaufwand für diese Prozedur, ist der beschriebene letzte Schritt der Plasmidpräparation besonders fehleranfällig. Ein teilweiser oder kompletter Verlust des DNA-Pellets tritt auch hin und wieder auf. Eine entscheidende Rolle scheint hierbei auch die Art (Material) des verwendeten Zentrifugationsgefäßes zu spielen.

Die ebenfalls beschriebene Anwendung von chaotropen Salzen und anschließender Bindung der Nukleinsäuren an Silikamembranen ist ebenfalls zeitaufwendig, zudem besteht durch das Einbringen chaotroper Salze in die Präparation die Gefahr einer Kontamination der letztlich isolierten DNA.

Die beschriebene Filtration alkoholischer Präzipitate im Kleinmaßstab besitzt den Nachteil, daß sie nicht linear auf den Großmaßstab übertragbar ist. Im Stand der Technik eingesetzte Membranen erlauben nur die Isolierung kleiner Mengen an Nukleinsäuren, da sich die Membranen rasch mit Nukleinsäuren sättigen und nichts mehr aufnehmen. Beim Entfernen des Präzpitatpuffers und beim Waschen geht daher häufig ein großer Teil der Nukleinsäuren wieder verloren. Um diesen Verlust zu vermeiden, ist die Erfindung gerichtet auf ein Verfahren zur Fällung von Plasmiden mit den folgenden Schritten
- Bereitstellen eines Isoliergefäßes mit zumindest einer darin angeordneten Membran;
- Beschicken des Isoliergefäßes mit einer Plasmide enthaltenden Lösung;
- Präzipitation der in der Lösung enthaltenen Plasmide mit Alkohol, so dass sich die Plasmide an die zumindest eine Membran binden,
**dadurch gekennzeichnet, dass** die Porengröße der zumindest einen Membran größer oder gleich 0,45 Mikrometer beträgt und keine Zentrifugationsschritte durchgeführt werden, dass die Membran eine hydrophile oder hydrophilisierte Membran ist, und dass die Membran aus Celluloseacetat oder Cellulosenitrat besteht.

Als Alkohole kommen für die Durchführung des erfindungsgemäßen Verfahrens zunächst alle Hydroxylderivate von aliphatischen oder acyclischen gesättigten oder ungesättigten Kohlenwasserstoffen in Betracht.

Unter den vorgenannten Hydroxylverbindungen werden die C1-C5-Alkanole - wie Methanol, Ethanol, n-Propanol, n-Butanol, tert.-Butanol, n-Pentanol oder Mischungen derer bevorzugt. Besonders bevorzugt wird Isopropanol zur Durchführung des erfindungsgemäßen Verfahrens verwendet.

Dabei kann der Alkohol vor oder nach dem Beschicken des Isoliergefässes mit der Nukleinsäure enthaltenden Lösung mit dieser Lösung gemischt werden. Das Volumenverhältnis von Nukleinsäure enthaltender Lösung zu Alkohol, insbesondere Isopropanol, beträgt vorzugsweise 2:1 bis 1:1, besonders bevorzugt 1,67:1 bis 1:1 und beispielsweise 1,43:1.

Die Fläche der Membran ist vorzugsweise so gewählt, daß die gesamten in der Lösung enthaltenden Nukleinsäuren an die Membran binden können.

Die Erfindung ist auch gerichtet auf die Verwendung von Membranen aus Celluloseacetat oder Cellulosenitrat mit einer Porengröße von größer oder gleich 0.45 µm zur Bindung von Alkohol-gefällten Plasmiden, wobei keine Zentrifugationsschritte durchgeführt werden.

Als besonders vorteilhaft wird die Verwendung eines 0,45 µm Celluloseacetat- bzw. Cellulosenitratfilters bzw. die Verwendung mehrer Lagen von 0,65 µm Celluloseacetat- oder Cellulosenitratfilter angesehen. Die Prozedur ist sowohl als Vakuumfiltration, als auch als Druckfiltration anwendbar.

Das erfindungsgemäße Verfahren ermöglicht eine zeitsparende Überführung von Nukleinsäuren aus einem Hochsalzpuffersystem in ein Niedrigsalzpuffersystem, die ohne großen apparativen Aufwand möglich ist. Sie ist als Ersatz für die klassische alkoholische Fällung von DNA aus einem Hochsalzpuffer geeignet, die typischerweise mit Hilfe von Zentrifugationsschritten durchgeführt wird. Durch den hohen Wirkungsgrad der Methode (geringer Ausbeuteverlust) ist sie insbesondere für den präparativen Großmaßstab geeignet. Weiterhin werden durch das erfindungsgemäße Verfahren keine Fremdsubstanzen in die bereits aufgereinigte Nukleinsäuren eingeführt. Zudem ist die Fehleranfälligkeit gegenüber der klassischen Methode geringer (Verlust des Zentrifugationssediments während des Waschschritts ist hier nicht möglich).

Vorzugsweise erfolgt bei dem oben erläuterten Verfahren das Beschicken von oben. Grundsätzlich stehen verschiedenste Methoden zur Verfügung, die verschiedenen Lösungen, also plasmidhaltiger Immobilisierungspuffer, Waschpuffer, Eluat, etc, durch die Membranen hindurchzuführen.

Es können dies sein Gravitation, Vakuum, Überdruck (auf der Beschickungsseite), und Kapillarkräfte.

Zwischen dem Immobilisierungs- und dem Abloseschritt kann ein Waschen der immobilisierten Nukleinsäuren mit zumindest einem Waschpuffer erfolgen. Das Waschen umfasst für jeden Waschpuffer vorzugsweise folgende Schritte:
- Aufbringen einer vorbestimmten Menge an Waschpuffer auf die Oberfläche, und
- Hindurchführen des Waschpuffers durch die Oberfläche.

Das Beschicken und Immobilisieren der Nukleinsäuren kann wiederum folgende Schritte umfassen:
- Mischen der Plasmidprobe mit einem Immobilisierungspuffer,
- Beschicken der Plasmidprobe mit dem Immobilisierungspuffer auf die Oberfläche, und
- Hindurchführen der füssigen Bestandteile durch die Oberfläche in im wesentlichen der Richtung der Beschickung.

Das Verfahren weist den großen Vorteil auf, leicht automatisierbar zu sein, so daß zumindest einer der Schritte durch einen Automaten vollautomatisch durchgeführt werden kann. Ebenso ist es möglich, daß alle Schritte des Verfahrens durch einen Automaten in gesteuerter Abfolge durchgeführt werden.

Speziell in diesen Fällen, aber auch bei manueller Bearbeitung ist es möglich, daß eine Mehrzahl von Plasmiden gleichzeitig der Isolierung unterworfen wird. Beispielsweise können Multi-Isoliergefässe in der Form marktüblicher "Multi-Well"-Gefässe mit 8, 12, 24, 48, 96 oder mehr einzelnen Isoliervertiefungen verwendet werden.

Das Abnehmen der Plasmide kann in zwei grundsätzlich verschiedene Richtungen erfolgen. Zum einen ist es möglich, die abgenommenen (eluierte) Plasmide durch die Membran durchzuschleusen (hindurchzuführen) und nach der Seite der Membran abzunehmen, die gegenüber der Seite liegt, auf welche die Plasmid-haltige Lösung bzw. das Lysat zugegeben wurde.

In diesem Fall wird des Plasmid in Richtung der Zugabe durch die Membran hindurch abgenommen. Die andere Möglichkeit besteht darin, die Plasmide von der Membran bzw. der Oberfläche auf der Seite der Zugabe abzunehmen. Die Abnahme erfolgt dann in Gegenrichtung der Zugabe oder "zur selben Richtung hin", in der zugegeben wurde; also auf der Seite der Zugabe. In diesem Fall müssen die Plasmide die Membran also nicht passieren. Bei einigen der erfindungsgemäßen Verfahren erfolgt das Abnehmen der Plasmide stets durch die Membran hindurch in Richtung der Zugabe.

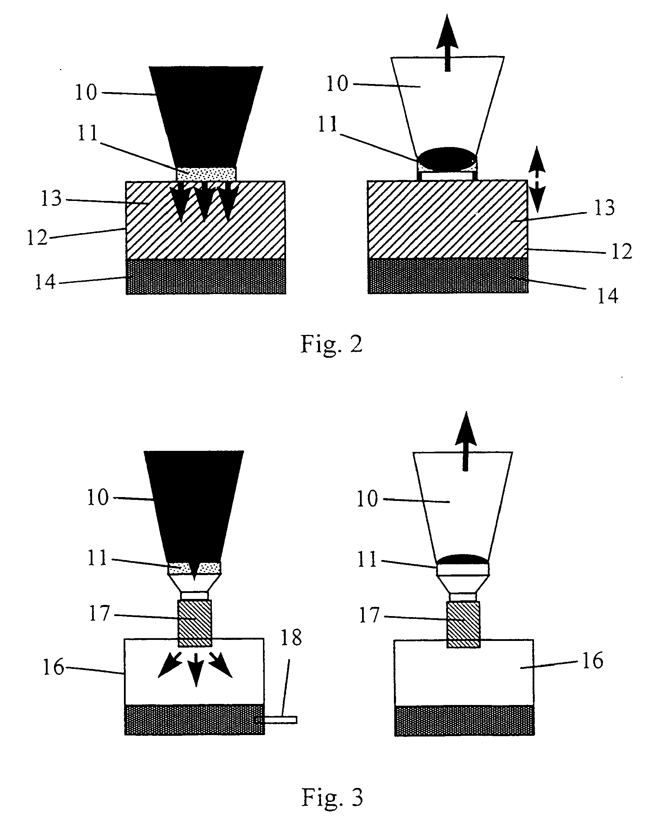

Werden die Plasmide von der Oberfläche im wesentlichen in Gegenrichtung der Richtung eluiert (abgelöst), in der sie aufgetragen und immobilisiert worden ist, so ist unter "derselben Richtung" dabei im Grunde genommen jede Richtung unter einem Winkel von kleiner oder gleich 180°, bezogen auf die Richtung der Zugabe, zu verstehen, so daß bei der Eluierung die Plasmide jedenfalls nicht die Membran, durchdringen, sondern in Gegenrichtung der Beschickungsrichtung von der Oberfläche entfernt werden, in der sie auf die Oberfläche aufgebracht worden sind. In bevorzugten Ausführungsformen werden demgegenüber die anderen Puffer, also derjenige Puffer, in dem sich die Plasmide beim Beschicken befinden, und ggfs. ein Waschpuffer, durch die Oberfläche durchgesaugt oder sonstwie transferiert. Wenn die Isolierung an einer in einem Gefäß befindlichen Membran erfolgt, wobei die Membran den gesamten Querschnitt des Gefässes ausfüllt, ist die bevorzugte Beschickung von oben. Der Abnahmeschritt erfolgt in diesem Fall wiederum nach oben. Fig. 2 zeigt beispielsweise ein trichterförmiges Isoliergefäss, das von oben beschickt wird und bei dem die Abnahme der Nukleinsäuren nach oben erfolgt.

Es versteht sich jedoch, daß auch bei Abnahme in Gegenrichtung der Zugabe andere Anordnungen denkbar sind, so z. B. eine Abnahme der Plasmide von unten. Es ist beispielsweise vorstellbar, daß ein Plasmide enthaltender Puffer wie ein Lysatpuffer mittels einer Saugvorrichtung aus einem Reaktionsgefäß unmittelbar in ein Isoliergefaß gesaugt wird, so daß sich die Plasmide an die Unterseite einer Membran in dem Isoliergefäß binden. In einem solchen Fall könnte die Abnahme der Plasmide von der Oberfläche dadurch erfolgen, daß ein Elutionspuffer von unten aufgesaugt wird und nach Ablösen der Plasmide wiederum nach unten in ein Gefäss abgelassen wird. Hierbei erfolgt also die Abnahme der Plasmide nach unten.

Auch eine seitliche Abnahme der Plasmide ist möglich, beispielsweise wenn eine flachliegende Säule mit einer darin angeordneten Membran im Durchflußverfahren mit einem Lysat beschickt wird und im Anschluß die liegende Säule auf der Seite der Membran, an der die Plasmide binden, mit Eluierpuffer gespült wird.

Ein Beispiel für den maximal möglichen Winkel von 180° ist eine Schräge mit einer zur Bindung von Plasmiden geeigneten Oberfläche, über welche die verschiedenen Lösungen bzw. Puffer herabfließen. Wie alle Puffer, kommt auch der Eluierpuffer von einer Seite und fließt zur anderen Seite ab. In diesem Fall bilden Einströmrichtung des Puffers und Abströmrichtung des Puffers mit den darin aufgenommenen Plasmiden einen Winkel von 180°, die Abnahme erfolgt jedoch immer noch auf derselben Seite der Oberfläche wie die Immobilisierung.

Nach dem erfindungsgemäßen Verfahren nimmt man die oben beschriebene, Plasmide enthaltende Probe in einer Lösung auf, die geeignete Salze und/oder Alkohol(e) enthält, schließt anschliessend ggf. den Ansatz auf und führt die so erhaltene Mischung mittels eines Vakuums, mittels Überdruck, durch Kapillarkräfte oder durch andere geeignete Verfahren durch eine poröse Membran hindurch, wobei die Plasmide an der Membran immobilisiert werden.

Als Salze für das Immobilisieren von Nukleinsäuren an Membranen oder anderen Oberflächen und/oder für die Lyse von Nukleinsäureproben kommen Salze von Metallkationen, wie Alkali- oder Erdalkalimetallen, mit Mineralsäuren in Frage; insbesondere Alkali- oder Erdalkalihalogenide bzw. -sulfate oder -phosphate, worunter die Halogenide des Natriums, Lithiums oder Kaliums oder Magnesiumsulfat besonders bevorzugt werden. Auch andere Metallkationen, beispielsweise Mn, Cu, Cs oder Al, oder das Ammoniumkation können verwendet werden, bevorzugt als Salze von Mineralsäuren.

Ferner sind zur Durchführung des erfindungsgemäßen Verfahrens Salze von ein- oder mehrbasigen oder auch polyfunktionellen organischen Säuren mit Alkali- oder Erdalkalimetallen geeignet. Darunter fallen insbesondere Salze des Natriums, des Kaliums oder des Magnesiums mit organischen Dicarbonsäuren - wie z. B. Oxal-, Malon- oder Bernsteinsäure - oder mit Hydroxy- bzw. Polyhydroxycarbonsäuren - wie z. B. bevorzugterweise mit Zitronensäure.

Die oben angegebenen Substanzen zur Immobilisierung der Nukleinsäuren auf den Oberflächen und/oder zur Lyse von Nukleinsäureproben können dabei einzeln oder auch in Mischungen verwendet werden, wenn sich diese als für bestimmte Anwendungen geeigneter erweisen.

Als besonders zweckmäßig hat sich dabei der Einsatz von sog. chaotropen Agenzien herausgestellt. Chaotrope Substanzen sind in der Lage, die dreidimensionale Struktur der Wasserstoffbrückenbindungen zu stören. Hierdurch werden auch die intramolekularen Bindungskräfte geschwächt, die bei der Ausbildung der räumlichen Strukturen, wie z. B. Primär-, Sekundär-, Tertiär- oder Quartärstrukturen, bei biologischen Molekülen beteiligt sind. Geeignete chaotrope Agenzien sind dem Fachmann aus dem Stand der Technik hinlänglich bekannt [Römpp, Lexikon der Biotechnologie, Herausgeber H. Dellweg, R.D. Schmid u. W.E. Fromm, Thieme Verlag, Stuttgart 1992].

Als bevorzugte chaotrope Substanzen gelten gemäß der vorliegenden Erfindung beispielsweise Salze aus der Gruppe der Trichloracetate, Thiocyanate, Perchlorate, Iodide oder Guanidinium-Hydrochlorid und Harnstoff.

Die chaotropen Substanzen werden dabei in 0,01 bis 10 molarer wässeriger Lösung, bevorzugt in 0,1 bis 7 molarer wässeriger Lösung und besonders bevorzugt in 0,2 bis 5 molarer wässeriger Lösung eingesetzt. Hierbei können die vorbezeichneten chaotropen Agenzien allein oder in Kombinationen verwendet werden. Insbesondere werden 0,01 bis 10 molare wässerige Lösungen, bevorzugt 0,1 bis 7 molare wässerige Lösungen und besonders bevorzugt in 0,2 bis 5 molare wässerige Lösungen von Natriumperchlorat, Guanidinium-Hydrochlorid, Guanidinium-isothiocyanat, Natriumiodid und/oder Kaliumiodid eingesetzt.

Die in den erfindungsgemäßen Verfahren zur Lyse, zur Bindung, zum Waschen und/oder zur Elution eingesetzten Salzlösungen sind vorzugsweise gepuffert. Als Puffersubstanzen kommen alle geeigneten Puffersysteme, wie beispielsweise Karbonsäure-Puffer, insbesondere, Citrat-Puffer, Acetat-Puffer, Succinat-Puffer, Malonat-Puffer sowie Glycin-Puffer, Morpholinopropansulfonsäure (MOPS) oder Tris(hydroxymethyl)aminomethan (Tris) in einer Konzentration von 0,001 - 3 Mol/Liter, bevorzugt 0,005 - 1 Mol/Liter, besonders bevorzugt 0,01 - 0,5 Mol/Liter, insbesondere bevorzugt 0,01 - 0,2 Mol/Liter.

Als Alkohole kommen für die Durchführung des erfindungsgemäßen Verfahrens zunächst alle Hydroxylderivate von aliphatischen oder acyclischen gesättigten oder ungesättigten Kohlenwasserstoffen in Betracht. Dabei ist es zunächst unerheblich, ob diese Verbindungen jeweils eine, zwei, drei oder mehr Hydroxylgruppen - wie mehrwertige C1-C5-Alkanole, beispielsweise Ethylenglykol, Propylenglykol oder Glycerin - enthalten.

Daneben zählen ebenfalls die Zuckerabkömmlinge, die sog. Aldite, wie auch die Phenole - beispielsweise Polyphenole - zu den erfindungsgemäß einsetzbaren Alkoholen.

Unter den vorgenannten Hydroxyverbindungen werden die C1-C5-Alkanole - wie Methanol, Ethanol, n-Propanol, tert.-Butanol und die Pentanole besonders bevorzugt.

Als hydrophil gelten im Sinne der vorliegenden Offenbarung solche Stoffe bzw. Membranen, die von ihrem chemischen Charakter her sich leicht mit Wasser mischen bzw. Wasser aufnehmen.

Als hydrophob gelten im Sinne der vorliegenden Offenbarung solche Stoffe bzw. Membranen, die von ihrem chemischen Charakter her nicht in Wasser eindringen - bzw. vice versa - und die nicht darin zu bleiben vermögen.

Eine Membran wird durch eine Folie aus einem polymeren Material gebildet. Das Polymer wird bevorzugt aus Monomeren mit polaren Gruppen aufgebaut.

Bei Verwendung hydrophober Membranen gelten als bevorzugt im Sinne der vorliegenden Offenbarung Membranen, die aus einem hydrophilen Grundmaterial bestehen und die durch eine entsprechende chemische Nachbehandlung, die an sich aus dem Stand der Technik bekannt ist, hydrophobisiert wurden, wie z. B. hydrophobisierte Nylon-Membranen, die kommerziell erhältlich sind.

Unter hydrophobisierten Membranen werden im sinne der vorliegenden Offenbarung allgemein solche Membranen verstanden, die als ursprünglich hydrophile Membran mit den unten erwähnten Hydrophobisierungsmitteln überzogen wurden. Derartige Hydrophobisierungsmittel überziehen hydrophile Substanzen mit einer dünnen Schicht hydrophober Gruppen, wozu beispielsweise längere Alkylketten oder Siloxangruppen gehören. Geeignete Hydrophobisierungsmittel sind aus dem Stand der Technik in großer Zahl bekannt und stellen erfindungsgemäß Paraffine, Wachse, Metallseifen etc., ggf. mit Zusätzen an Aluminium bzw. Zirkoniumsalzen, quartäre organische Verbindungen, Harnstoffderivate, fettstoffmodifizierte Melaminharze, Silicone, zinkorganische Verbindungen, Glutardialdehyde und ähnliche Verbindungen dar.

Daneben gelten als Offenbarungsgemäß einsetzbare hydrophobe Membranen solche Membranen, die per se hydrophob sind oder die hydrophobisiert sind und deren Grundmaterial polare Gruppen aufweisen kann. Gemäß dieser Kriterien eignen sich beispielsweise - insbesondere hydrophobisierte - Materialien aus der folgenden Gruppe für den beschriebenen Einsatz:

Nylon, Polysulfone, Polyethersulfone, Cellulosenitrat, Polyproyplen Polycarbonate, Polyacrylate sowie Acrylatcopolymere, Polyurethane, Polyamide, Polyvinylchlorid, Polyfluorcarbonate, Polytetrafluorethylen, Polyvinylidendifluorid, Polyethylentetrafluorethylen-Copolymerisate, Polyethylenchlortrifluorethylen-Coplymerisate oder Polyphenylensulfid sowie Cellulose und CelluloseMischester, Celluloseacetat oder Nitrocellulosen wie auch Polybenzimidazole, Polyimide, Polyacrylnitrile, Polyacrylnitril-Copolymere, hydrophobisierte Glasfasermembranen, worunter hydrophobisierte Nylon-Membrane besonders bevorzugt sind.

Bevorzugte hydrophile Oberflächen umfassen per se hydrophile Materialien und auch hydrophobe Materialien, die hydrophilisiert worden sind. Beispielsweise können verwendet werden hydrophiles Nylon, hydrophile Polyethersulfone, hydrophile Polycarbonate, hydrophile Polyester, hydrophile Polytetrafluorethylene auf Polypropylengeweben, hydrophile Polytetrafluorethylene auf Polypropylenvliesen, hydrophilisierte Polyvinylidendifluoride, hydrophilisierte Polytetrafluorethylene, hydrophile Polyamide, Nitrocellulose, hydrophile Polybenzimidazole, hydrophile Polyimide, hydrophile Polyacrylnitrile, hydrophile Polyacrylnitril-Copolymere, hydrophiles Polypropylen, Cellulosenitrat, Cellulosemischester und Celluloseacetat.

Die oben geschilderten Membranen sind in ihrer Anwendung bei Nukleinsäurenbindung zum Teil aus dem Stand der Technik vorbekannt, wenn auch nicht im erfindungsgemäßen Kontext. Eine Reihe von Materialien ist jedoch aus dem Stand der Technik nicht für diese Verwendung bekannt.

Im Falle der Fällung von Nukleinsäuren mit Isopropanol nach dem oben angegebenen Verfahren muß die Porengröße über 0,45 µm betragen.

Als Waschpuffer kommen ebenfalls die oben beschriebenen Salze oder Alkohole bzw. Phenole oder Polyphenole in Frage. Auch Detergenzien und Naturstoffe im weitesten Sinne, wie etwa Albumin oder Milchpulver, können für die Waschschritte verwendet werden. Die Zugabe chaotroper Substanzen ist ebenfalls möglich. Polymere können ebenso wie lösende Detergentien und ähnliche Stoffe zugegeben werden. Der Waschpuffer und die darin enthaltenen Substanzen sollten jedenfalls allgemein in der Lage sein, unerwünschte Verunreinigungen zu binden, zu solubilisieren, oder mit ihnen zu reagieren, so daß diese Verunreinigungen oder ihre Abbauprodukte zusammen mit dem Waschpuffer entfernt werden können.

Die Temperaturen liegen im Waschschritt in einem Intervall von üblicherweise 10° bis 30 °C, vorzugsweise bei Raumtemperatur, wobei auch höhere oder niedrigere Temperaturen erfolgreich angewandt werden können. So bietet es sich beispielsweise an, bei einer Elution bei niedrigen Temperaturen, z.B. 2°C, auch den Waschpuffer zu kühlen, um die Temperatur des Isoliergefässes und der Oberfläche bzw. Membran auf den gewünschten Temperaturwert vorzukühlen. Eine Anwendung für niedrige Temperaturen ist die cytoplasmatische Lyse, bei der die Zellkerne zunächst unbeschädigt bleiben. Höhere Temperaturen des Waschpuffers bewirken auf der anderen Seite eine bessere Solubilisierung der auszuwaschenden Verunreinigungen.

Zur Elution der gebundenen Plasmide eignen sich erfindungsgemäß Wasser oder wässerige Salzlösungen als Elutionsmittel. Als Salzlösungen werden Pufferlösungen eingesetzt, die aus dem Stand der Technik bekannt sind, wie beispielsweise Morpholinopropansulfonsäure (MOPS), Tris(hydroxymethyl)aminomethan (TRIS), 2-[4-(2-Hydroxyethyl)-1-piperazino]ethansulfonsäure (HEPES) in einer Konzentration von 0,001 bis 0,5 Mol/Liter, bevorzugt 0,01 bis 0,2 Mol/Liter, besonders bevorzugt 0,01 bis 0,05 molare Lösungen. Daneben werden bevorzugt wässerige Lösungen von Alkali- oder Erdalkalimetallsalzen, insbesondere deren Halogenide, eingesetzt, darunter 0,001 bis 0,5 molare, bevorzugt 0,01 bis 0,2 molare, besonders bevorzugt 0,01 bis 0,05 molare wässerige Lösungen von Natriumchlorid, Lithiumchlorid, Kaliumchlorid oder Magnesiumdichlorid. Daneben können bevorzugt auch Lösungen von Salzen der Alkalimetalle oder Erdalkalimetalle mit Carbon- oder Dicarbonsäuren wie Oxalsäure oder Essigsäure, wie Lösungen von Natriumacetat oder -oxalat in Wasser eingesetzt werden, beispielsweise in dem zuvor genannten Konzentrationsbereich, wie z. B. 0,001 bis 0,5 molar, bevorzugt 0,01 bis 0,2 molar, besonders bevorzugt 0,01 bis 0,05 molar.

Auch die Zugabe von Hilfsstoffen wie Detergenzien oder DMSO ist möglich. Soll mit den eluierten Nukleinsäuren eine chemische Reaktion durchgeführt werden, sei es unmittelbar an der Membran oder in einem weiteren Reaktionsgefäß, ist es auch möglich, solche Substanzen oder andere Hilfsstoffe dem Elutionspuffer zuzugeben, die in den verwendeten Reaktionsansätzen verwendet werden sollen. So ist beispielsweie die Zugabe von DMSO in niedrigen Konzentrationen bei vielen Reaktionsansätzen üblich.

Nach einer an Nukleinsäuren durchgeführten chemischen Reaktion können diese auch mit dem Reaktionspuffer eluiert werden. Beispielsweise kann die Nukleinsäure nach einer SDA- oder einer NASBA-Reaktion mit dem Reaktionspuffer oder dem Reaktions-Master-Mix eluiert werden.

Ganz besonders wird reines Wasser als Elutionsmittel bevorzugt, beispielsweise demineralisiertes, bidestiliertes, oder ultrareines Millipore-Wasser.

Die Eluierung kann bei Temperaturen von unter 0°C bis 90°C, bespielsweise bei 10° bis 30°C oder auch bei höheren Temperaturen erfolgreich durchgeführt werden. Auch ein Eluieren mit Wasserdampf ist möglich. Die untere Grenze der Elutionstemperatur ist beim Gefrierpunkt des Elutionspuffers zu sehen.

Als Isoliergefäß kann ein Reaktionsgefäß verwendet werden, das eine Membran enthält. Diese kann in Kontakt mit einem absorbierenden Material, wie einem Schwamm, gebracht werden, um die verschiedenen, verwendeten Puffer durch die Membran durchzusaugen. Der Schwamm funktioniert daher wie die Kombination einer Vakuumpumpe in Verbindung mit einem Abfallbehältnis. Zur Gewinnung des Eluats wird der Kontakt des absorbierenden Materials mit der Membran unterbunden, so daß das Eluat nicht verloren gehen kann, sondern abgenommen oder/und weiter untersucht werden kann.

Das Isoliergefäß zur Isolierung von Plasmiden ist mit zumindest einem zylinderförmigen Oberteil mit einer oberen Öffnung, einer unteren Öffnung und einer Membran versehen, die an der unteren Öffnung angeordnet ist und den gesamten Querschnitt des Oberteils ausfüllt; einem Unterteil mit einem absorbierenden Material; und einem Mechanismus zur Verbindung zwischen Oberteil und Unterteil, wobei bei hergestellter Verbindung die Membran in Kontakt mit dem absorbierenden Material ist und bei nichthergestellter Verbindung die Membran nicht in Kontakt mit dem absorbierenden Material ist.

Vorzugsweise ist das Unterteil ein Zylinder gleichen Durchmessers wie das Oberteil. Auf diese Weise erhält man ein einfaches Röhrchen vom im wesentlichem konstanten Durchmesser, das wie herkömmliche Reaktionsgefässe handhabbar ist. Speziell wenn das Oberteil oder Oberteil plus Unterteil ein Röhrchen ist bzw. bilden, welches in Reaktionsgefäßhalter einstellbar ist, wie sie in Laboratorien Verwendung finden, ist dieser Effekt erzielbar. Der Mechanismus kann ein Anschluß sein, der eine räumliche Trennung von Oberteil und Unterteil erlaubt, beispielsweise ein Bajonettanschluß, ein Steckanschluß oder ein Schraubanschluß. Ein Eajonettanschluß hat dabei den Vorteil, leichter ver- und entriegelbar zu sein, während der Schraubverschluß die bessere, wasserdichtere Verbindung von. Ober- und Unterteil erlaubt. Alternativ kann auch eine Sollbruchstelle zwischen Ober- und Unterteil vorgesehen sein, die zumindest die einmalige Trennung der beiden Teile gestattet und die besonders preisgünstig herstellbar ist.

Der Mechanismus kann allerdings auch ein Schieber sein, der zwischen absorbierendem Material und Membran einschiebbar ist. Auch durch diese Ausführung kann eine Trennung von Membrann und absorbierenden Material erreicht werden.

Um die Verarbeitungskapazität zu erhöhen und das erfindungsgemäße Verfahren noch ökonomischer ausführen zu können, bietet sich desweiteren an, das oben beschriebene Isoliergefäß so zu modifizieren, daß mehrere Oberteile auf einem Unterteil angeordnet sind. Das Unterteil kann dann gleichzeitig als Halter der Anordnung dienen und zudem so dimensioniert sein, daß eine Vielzahl von Isolierungsvorgängen, jedenfalls mehr als Anschlüsse für Oberteil im Unterteil vorhanden sind, durchgeführt werden kann, bevor die Saugkapazität des absorbierenden Materials im Unterteil erschöpft ist.

Das absorbierende Material im Unterteil kann einen Schwamm oder/und ein Granulat aufweisen. Das Granulat kann beispielsweise aus superabscrbierendem Material bestehen, wie es dem Fachmann auf dem Gebiet der Absorptionstechnik (z.B. bei Hygieneartikeln) geläufig ist.

Hinsichtlich der einzelnen Schritte wird das erfindungsgemäße Verfahren allgemein typischerweise wie folgt durchgeführt:

Wenn von biologischen Proben ausgegangen wird, sind diese zunächst in einem geeigneten Puffer zu lysieren. Hierbei können weitere Verfahren zur Lyse zum Einsatz kommen, beispielsweise eine mechanische Einwirkung, wie Homogenisierung oder Ultraschall, enzymatische Einwirkung, Temperaturveränderug oder Additiva. Falls erforderlich oder gewünscht, kann sich an diese Lyse ein Vorreinigungsschritt anschliessen, um Debris aus dem Lysat zu entfernen. Im Anschluß daran werden, falls noch nicht geschehen, die Bedingungen im Lysat eingestellt, unter denen eine Immobilisierung der Plasmide an die Membran erfolgen kann. Auch nach Einstellung der Bindebedingungen kann sich, kumulativ oder alternativ zur obigen Vorreinigung, ein Vorreinigungsschritt anschliessen.

Dieses vorbehandelte Lysat der zur Gewinnung der Plasmide dienenden Probe oder dass die ursprünglich freie(n) Plasmid(e) (falls nicht von einer biologischen Probe ausgegangen wird) wird/werden beispielsweise in eine (Plastik-)Säule pipettiert, in der - beispielsweise auf dem Boden - die Membran fixiert wird. Zweckmäßigerweise kann die Membran auf einer Fritte fixiert werden, die als mechanische Unterstützung dient. Anschließend wird das Lysat durch die Membran geführt, was durch Anlegen eines Vakuums am Ausgang der Säule erreicht werden kann. Auf der anderen Seite kann der Transport durch einen Lysat-seitigen Überdruck erfolgen. Daneben kann - wie schon zuvor erwähnt - der Lysattransport durch die Einwirkung von Kapillarkräften bewerkstelligt werden; letzteres kann zum Beispiel mit einem schwammartigen Material geschehen, das unterhalb der Membran mit dem Lysat bzw. Filtrat in Kontakt gebracht wird.

Der in bevorzugten Ausführungsformen eingefügte Waschschritt kann erfolgen, indem der Waschpuffer durch die Membran hindurchtransportiert wird oder auf derselben Seite der Oberfläche verbleibt wie die Plasmide. Wird der Waschpuffer hindurchtansportiert bzw. gesaugt, so kann dies auf unterschiedliche Weisen geschehen, z. B. durch einen auf der anderen Seite der Membran angeordneten Schwamm, eine Saug- oder Überdruckvorrichtung oder durch Gravitation.

Der Vorteil einer Anordnung mit einem absorbierenden, beispielsweise schwammartigen Material besteht in einer einfachen, sicheren und bequemen Möglichkeit der Filtrat-Entsorgung - es muß in diesem Fall nur der Schwamm, der nunmehr mit dem Filtrat mehr oder weniger vollgesogen ist, ausgetauscht werden. Es wird an dieser Stelle deutlich, daß die Säule kontinuierlich oder auch batch-weise betrieben werden kann und daß beide Betriebsarten vollautomatisiert durchgeführt werden können, bis die Membran mit Plasmid gesättigt ist. In letzten Schritt erfolgt ggfs. die Elution des Plasmids, welches beispielsweise von der Membran abpipettiert bzw. abgehoben oder in sonstiger Weise nach oben entfernt werden kann, wenn keine In-situ Analyse der noch gebundenen Plasmide werden soll.

Die gewünschten Plasmide fallen in schwach oder nichtsalzhaltigen Lösungen in sehr kleinen Volumina an, was von großem Vorteil für alle molekularbiologischen Analyseverfahren ist, da hier gewünscht ist, reine Nukleinsäuren in möglichst kleinen Volumina bei gleichzeitig hoher Konzentration einzusetzen. Um möglichst kleine Volumina an Eluat erzielen zu können, werden als Oberflächen insbesondere Membranen bevorzugt, die möglichst dünn sind, so daß sich nur wenig Flüssigkeit in ihnen ansammeln kann.

Ferner bietet die vorliegende Erfindung den Vorteil, daß bei einer vertikalen Anordnung des Gefässes (Membran dann horizontal orientiert) das über der Membran befindliche Volumen als Reaktionsraum genutzt werden kann. So ist es z. B. möglich, nach dem Isolieren und Ablösen der nach dem erfindungsgemäßen Verfahren gewonnenen Plasmide, diese zunächst nicht abzunehmen, sondern im Isoliergefäß zu belassen und einer molekularbiologischen Applikation - wie Restriktionsverdauung, RT, PCR, RT-PCR, in vitro Transkription, NASBA, rolling circle, LCR (ligase chain reaction), SDA (strand displacement amplification) oder Enzymreaktionen wie RNase und DNase-Verdau zur vollständigen Entfernung der jeweils nicht erwünschten Nukleinsäuren, zu unterwerfen, die aus diesen Reaktionen hervorgehenden Nukleinsäuren gemäß dem erfindungsgemäßen Verfahren erneut an die Membran zu binden oder im Überstand zu belassen, ggfs. wie beschrieben zu waschen und anschließend zu eluieren, zu isolieren, bzw. zu analysieren, beispielsweise mittels Chromatographie, Spektroskopie, Fluorometrie, Elektrophorese oder ähnlichen Meßverfahren.

Die erfindungsgemäß isolierten Plasmide sind frei von nukleinsäureabbauenden Enzymen und haben eine derartig hohe Reinheit, daß sie unmittelbar in verschiedensten Weisen weiterbehandelt und bearbeitet werden können.

Die erfindungsgemäß hergestellten Plasmide könnten für Klonierungen verwendet werden und als Substrate für verschiedenste Enzyme dienen, wie beispielsweise DNA-Polymerasen, RNA-Polymerasen wie z.B. T7-Polymerase oder T3-Polymerase, DNA-Restrikticnsenzyme, DNA-Ligase, reverse Transkriptase und andere.

Die durch das erfindungsgemäße Verfahren bereitgestellten Plasmide eignen sich in besonders guter Weise zur Amplifikation, insbesondere für die PCR, Strand Displacement Amplifikation, Rolling Circle Verfahren, Ligase Chain Reaction (LCR), SunRise, NASBA und ähnlicher Verfahren.

Das erfindungsgemäße Verfahren eignet sich weiterhin in besonders guter Weise zur Bereitstellung von Plasmiden für die Verwendung in der Diagnostik, beispielsweise der Lebensmittelanalyse, bei toxikologischen Untersuchungen, in der medizinischen und klinischen Diagnostik, der Erreger-Diagnostik, der Genexpressionsanalyse und in der Umweltanalytik. Die Verfahren eignen sich insbesondere für ein Diagnoseverfahren, welches dadurch gekennzeichnet ist, daß die durch das erfindungsgemäße Verfahren gereinigten Plasmide in einem Folgeschritt amplifiziert werden und anschließend und/oder gleichzeitig die so amplifizierten Nukleinsäuren detektiert werden (z. B. Holland, P.M. et al., 1991. Proc. Natl. Acad. Sci. 88, 7276 - 7280. Livak, K.J. et al., 1995. PCR Methods Applic. 4, 357 - 362; Kievits, T. et al., 1991. J. Virol. Meth. 35, 273 - 286; Uyttendaele, M. et al., 1994. J. Appl. Bacteriol. 77, 694 - 701).

Im folgenden wird ein Beispiel für die Automatisierbarkeit des erfindungsgemäßen Verfahrens erläutert und werden Beispiele für die Durchführung des Verfahrens mit verschiedenen Membranen angegeben. Hierbei wird Bezug genommen auf die beigefügten Zeichnungen, in denen folgendes dargestellt ist.
Fig. 1 zeigt einen zur Durchführung des erfindungsgemäßen Verfahrens geeigneten Automaten in einer schematisierten Darstellung.
Fig. 2 zeigt eine erste Ausführungsform eines Isoliergefässes und Abfallbehälters zur Durchführung des erfindungsgemäßen Verfahrens.
Fig. 3 zeigt eine zweite Ausführungsform eines Isoliergefässes und Abfallbehälters zur Durchführung des erfindungsgemäßen Verfahrens.
Fig. 4 zeigt eine dritte Ausführungsform eines Isoliergefässes und Abfallbehälters zur Durchführung des erfindungsgemäßen Verfahrens.
Fig. 5 zeigt Ausführungsformen von Isoliergefässen mit Oberteil.

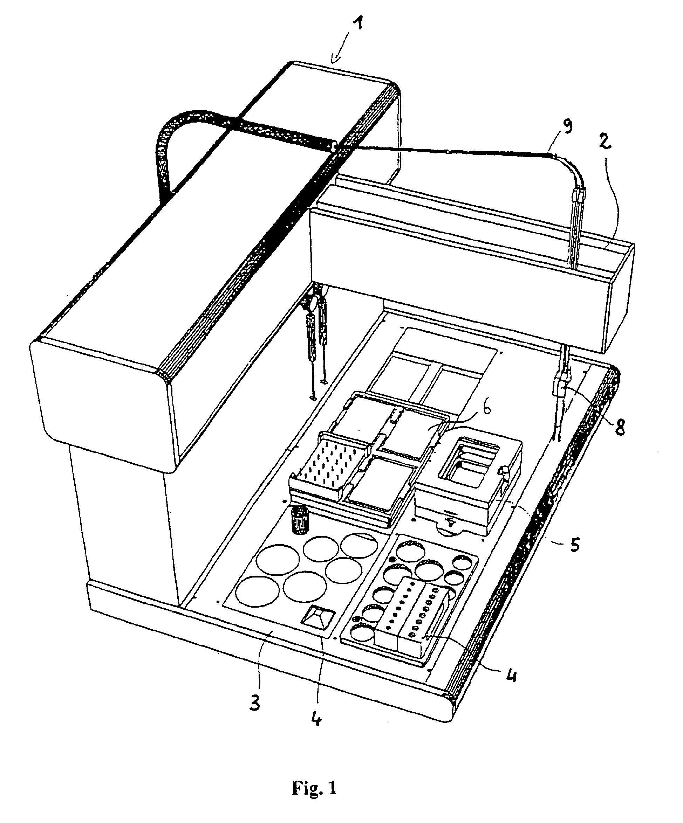

Das erfindungsgemäße Verfahren wird vorzugsweise teilweise oder vollständig, d.h. in allen Schritten, automatisiert durchgeführt. Ein Beispiel für einen geeigneten Automaten ist in Fig. 1 dargestellt, bei dem ein Hauptteil 1 mit Steuerungselektronik und Antriebsmotoren mit einer Arbeitsplattform 3 und einem fahrbaren Arm 2 ausgestattet ist. Auf der Arbeitsplattform sind verschiedene Elemente positioniert, so Bereiche 4 zur Halterung von verschiedenen Gefässen. Eine Absaugvorrichtung 5 dient zum Absaugen von Flüssigkeiten aus darüber positionierten, nach unten offenen Isoliergefässen, oder sonst mit der Absaugvorrichtung verbundenen Gefässen. Ein Rüttler 6 ist ebenfalls vorgesehen, der beispielsewise zum Lysieren von biologischen Proben verwendet werden kann. Die verwendeten Anordnungen von Isoliergefässen sind beispielsweise Spritzgußteile mit integrierten Isoliergefässen, in die Membranen eingelegt sind. Es können typischerweise 8, 12, 24, 48, 96 oder bis zu 1536 Isoliergefässe vorgesehen sein, wie sie z.B. in den Formaten moderner Multi-Well-Platten zur Verfügung gestellt werden. Auch noch höhere Isoliergefäßzahlen in einer Anordnung sind vorstellbar, wenn entsprechende Standards zur Verfügung stehen. Mit Hilfe von Luer-Adaptern ist es jedoch auch möglich, die Böden der Anordnungen separat bereitzustellen und je nach Bedarf mit einem oder mehreren Isoliergefässen zu bestücken. Auch einzeln gearbeitete Isoliergefässe ohne Luer-Adapter werden miterfasst.

Unter eine Saug- und Dispensiervorrichtung 8 werden die Anordnungen von Isoliergefässen in den Automaten eingesetzt und über diese können Flüssigkeiten aufgenommen und abgegeben werden. Hierbei können mehrere einzelne Saugrohre vorgesehen sein, um mehr als ein Isolier- oder Reakticnsgefäß gleichzeitig bearbeiten zu können. Die Saug- und Dispensiervorrichtung 8 erfüllt also die Funktion einer Pipette. Sog und Druck werden der Saug- und Dispensiervorrichtung 8 über Schläuche 9 vermittelt.

Zur Plasmidisolierung können beispielsweise Reaktionsgefässe mit Zellen in den Rüttler/Halter 6 eingesetzt werden, in die mit der Dispensiervorrichtung 8 Lysepuffer eingefüllt wird. Nach Mischen werden die Zelllysate in Isoliergefässe überführt. Der Lysepuffer wird daraufhin durch die Oberflächen in den Isoliergefässen durchgesaugt. Im Anschluß können die Oberflächen mit einem Waschpuffer gespült werden, um Reste der Zelllysate zu entfernen, wobei auch der Waschpuffer nach unten abgesaugt wird. Schließlich wird Elutionspuffer in die Isoliergefässe dispensiert und nach eventuellem nochmaligen Rütteln werden die abgelösten Plasmide nach oben entnommen und in Aufbewahrungsgefässe überführt.

Üblicherweise werden Wechselspitzen an der Saug- und Dispensiervorrichtung 8 verwendet, um eine Kontamination der Proben zu verhindern.

Die Fig. 2 bis 4 zeigen verschiedene schematische Beispiele für geeignete Isoliergefässe zur Verwendung in der vorliegenden Erfindung.

In Fig. 2 ist ein trichterförmiges Isoliergefäß 10 mit einer Oberfläche 11, beispielsweise einer Membran, versehen, die auf einen Auffangbehälter 12 aufgesetzt ist, der ein schwammartiges Material 13 enthält, das der Aufnahme von Lysepuffer und Waschpuffer dient. Unter dem schwammartigen Material 13 kann eine Schicht Superabsorbens 14 angeordnet sein, um die Saugleistung zu verbessern. Alternativ kann die Schicht 14 auch ein Material enthalten, welches Wasser chemisch umzusetzen vermag, beispielsweise Acrylat. Das Wasser wird dadurch ebenfalls dem Prozeß entzogen. In den Trichter wird Lysat oder eine sonstige Aufbereitung von Nukleinsäuren gegeben. Das schwammartige Material 13 saugt die aufgetragene Flüssigkeit durch die Membran 11 hindurch. Vor der Zugabe des Eluierpuffers wird der Schwamm etwas von der Membran beabstandet, beispielsweise durch eine im Auffangbehälter 12 angeordnete Mechanik (nicht dargestellt). So wird beim letzten Schritt verhindert, daß der Eluatpuffer auch durch die Membran 11 gesaugt wird. Dieser verbleibt vielmehr auf der Oberfläche (Fig. 2) und kann zusammen mit den Plasmiden nach oben entnommen werden. Mit dieser Anordnung wird die Absaugvorrichtung 5 im Automaten nicht benötigt.

Fig. 3 zeigt ein weiteres Beispiel eines Isoliergefässes, das über einen an seinem unteren Ende angeordneten Luer-Anschluß mittels eines Luer-Adapters 17 mit einem Auffangbehälter 16 verbunden ist, der in diesem Fall keinen Schwamm enthält, sondern mittels eines Stutzen 18 mit einer Absaugvorrichtung verbunden ist. Lyse- und Waschpuffer können hier also durch Anlegen eines Vakuums durch die Membran 11 durchgesaugt werden. Beim Auftragen des Eluatpuffers bleibt das Vakuum abgeschaltet, so daß sich das Eluat nach oben entnehmen lässt. Durch die Verwendung eines Luer-Anschlusses sind individuelle Isoliergefässe der Anordnung von Isoliergefässen entnehmbar. Es versteht sich jedoch, daß der Vakuumauffangbehälter auch mit fest angebrachten Isoliergefässen, beispielsweise Multi-Well Gefässen von 8, 12, 24, 48, 96 oder mehr Einzelgefässen kombinierbar ist.

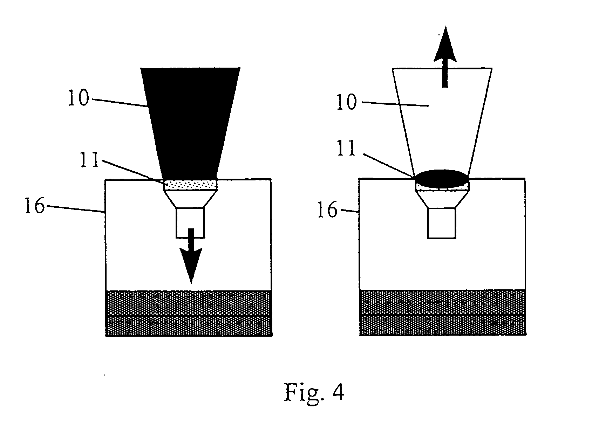

Fig. 4 zeigt schließlich eine Ausführungsform, bei der ein Auffangbehälter vorgesehen ist, in den die Puffer mittels Schwerkraft hineingesaugt werden. Der Eluatpuffer, der in geringem Volumen verwendet wird, hat kein hinreichendes Eigengewicht, um die Membran 11 zu durchdringen und kann wiederum nach oben abgenommen werden.

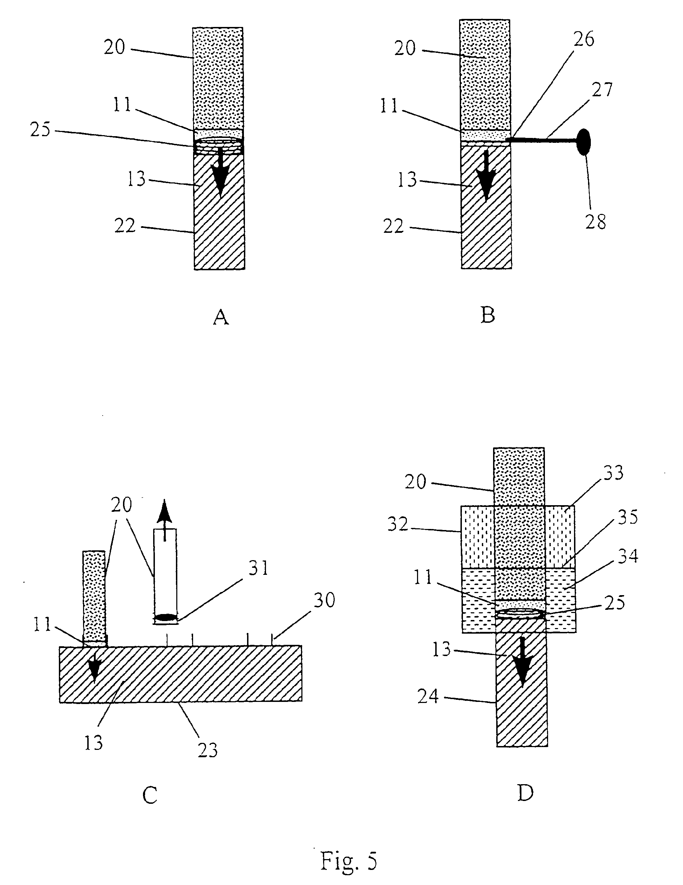

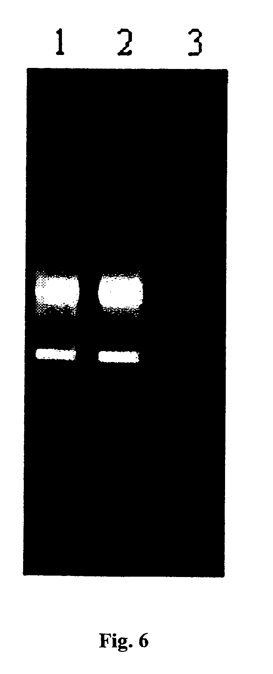

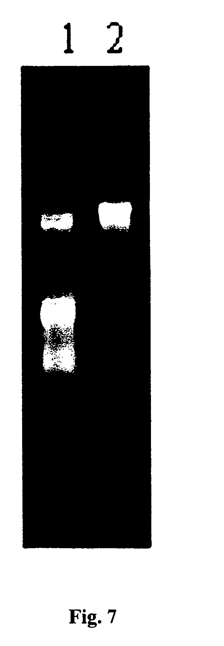

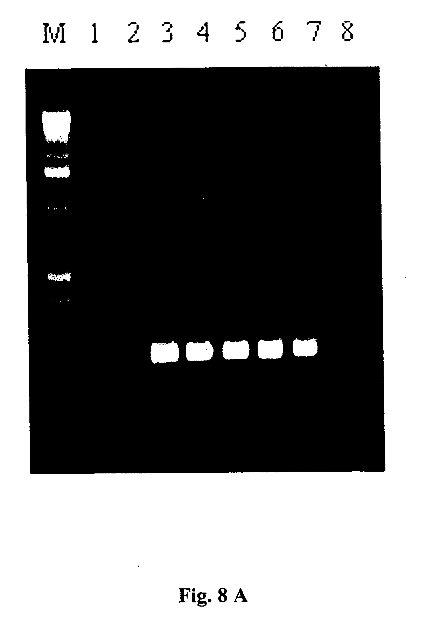

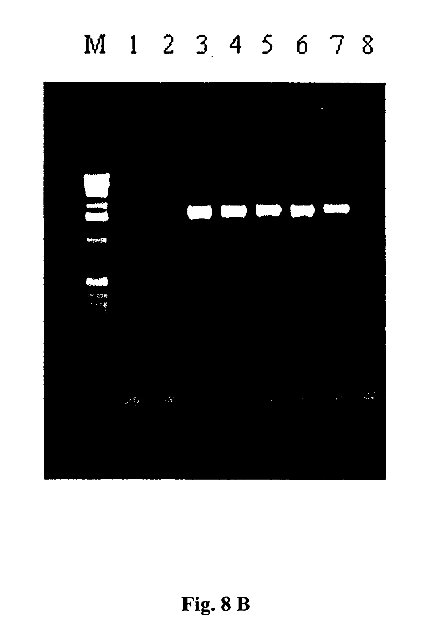

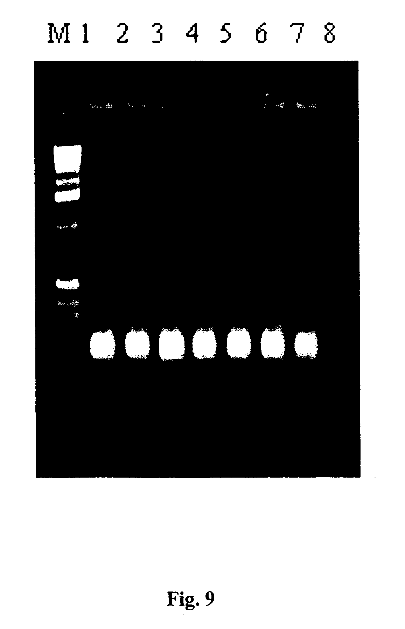

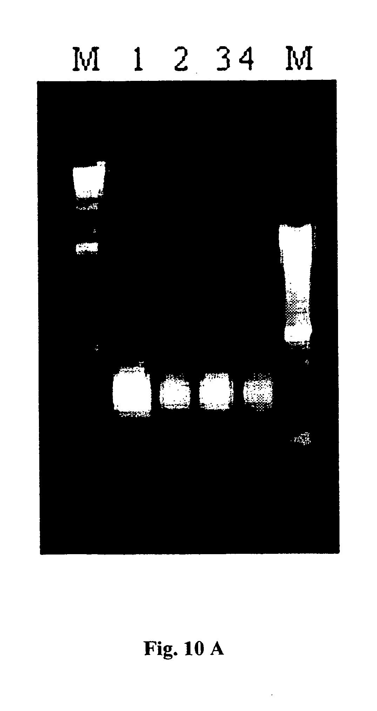

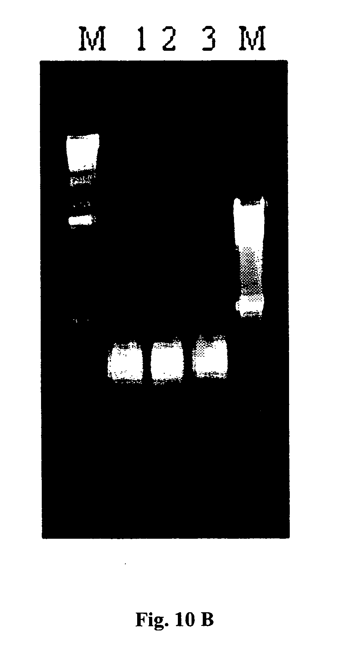

Fig. 5 zeigt Ausführungsformen der Isoliergefässe.

In Fig. 5 A ist ein Isoliergefäß mit einem zylinderförmigen Oberteil 20 dargestellt. Dieses Oberteil ist mittels eines Schraubanschlusses 25 mit einem Unterteil 22 verbunden. Anstelle des Schraubanschlusses können auch andere Verbindungsformen verwendet werden, sofern diese eine flüssigkeitsdichte Verbindung von Ober- und Unterteil zulassen und für ein Aufliegen der Membran 11 sorgen. Die Membran 11 ist in diesem Ausführungsbeispiel direkt an der unteren Öffnung des Oberteils 20 angebracht. Sie kann jedoch auch nach innen versetzt sein oder in einem anderen Winkel als 90° zur Oberteilwandung stehen. Das Unterteil weist ebenfalls eine zylinderförmige Form auf, kann jedoch in anderen Ausführungsformen anders ausgebildet sein. So könnte eine viereckige Form verwendet werden, welche die Standsicherheit des Oberteils 20 auf einem Untergrund verbessert. Eine Ausweitung des Unterteils 22 im Verhältnis zum Oberteil 20 ist ebenfalls möglich, beispielsweise falls in bestimmten Ausführungsformen des erfindungsgemäßen Verfahrens ein größerer Hohlraum im Unterteil 22 benötigt wird, um die verwendeten Lösungen vollständig in das absorbierende Material 13 aufnehmen zu können.

Eine zu der in Fig. 5 A gezeigten Auführungsform alternative Ausführungsform ist in Teil B dargestellt. Hier sind Oberteil 20 und Unterteil 22 fest miteinander verbunden bzw. können auch einstückig ausgeformt sein. Zwischen dem absorbierenden Material 13 und der Membran 11 kann ein Schieber 27 über eine Öffnung 26 in das Isoliergefäß eingeschoben werden, um Membran 11 und absorbierendes Material 13 voneinander trennen zu können. Am Schieber 27 ist in diesem Beispiel ein zusätzlicher Griffbereich 28 angeordnet, der ein einfaches Herausziehen des Schiebers 27 gestattet. Der Schieber kann jedoch auch ohne diesen Griffbereich ausgeführt sein. Wie in der Fig. 5 B gezeigt, expandiert das absorbierende Material 13 etwas, um den vom Schieber eingenommenen Raum überbrücken zu können und dadurch die Membran zu kontaktieren.

Fig. 5 C zeigt eine weitere Ausführungsform des Isoliergefäßes. Das Unterteil 23 ist hierbei mit mehreren Anschlüssen 30 zur Aufnahme von Oberteilen 20 ausgestattet, was die gleichzeitige Bearbeitung mehrerer Ansätze erlaubt. Die Oberteile 20 werden in diesem Beispiel mittels Schraubanschlüssen 31 mit dem Unterteil 23 verbunden. Obwohl in der Abbildung kleiner dargestellt als die Oberteile 20 der Fig. 5 A und B versteht es sich, daß die Oberteile von gleicher Größe (oder größer bzw. kleiner) sein können wie bzw. als in jenen Ausführungsformen.

Fig. 5 D zeigt schließlich ein Isoliergefäß mit einem die Membran 11 außen umgebenden Mantel 32 mit einem Kühlmittel. Oberteil 20 und Unterteil 24 sind in diesem Beispiel mittels eines Steckanschlusses miteinander verbunden. Eine andere Anschlußart oder eine einteilige Ausführung sind jedoch ebenso möglich. Der Mantel 32 besteht aus zwei Kompartimenten 33 und 34, die durch eine zerstörbare Trennwand 35 miteinander in Verbindung gebracht werden können. Beide Kompartimente 33, 34 sind mit Substanzen, beispielsweise Lösungen, gefüllt, bei deren Mischung nach Zerstörung der Trennwand 35 die Temperatur des Gemischs sinkt.

Die vorbeschriebene Erfindung wird durch die nachfolgenden Beispiele erläutert. Verschiedenartige, andere Ausgestaltungen des Verfahren werden für den Fachmann aus der vorstehenden Beschreibung und den Beispielen ersichtlich. Es wird jedoch ausdrücklich darauf hingewiesen, daß diese Beispiele und die diesen Beispielen zugeordnete Beschreibung lediglich der Erläuterung dienen und nicht als Einschränkung der Erfindung anzusehen sind.

### Beispiel 1

Restriktion von Plasmid-DNA mit dem Emzym AvaI auf einer hydrophoben Membran

Entsprechend dem Beispiel 1 werden Plastiksäulen mit hydrophoben Membranen (beispielsweise Supor-200 PR der Fa. Pall) hergestellt.
100 µl einer Plasmid enthaltenden wäßrigen Lösung (pCMVI der Fa. Clontech) werden mit 350 µl eines Guanidinium-Isothiocyanat enthaltenden Lysepuffers (4 M GITC, 0,1 M MgSO₄,
25 mM Natrium-Acetat, pH 4) vermischt. Anschließend werden 250 µl Isopropancl zugegeben und durch Auf- und Abpipettieren gemischt. Dieses Gemisch wird dann auf die Säule aufgetragen und entsprechend Beispiel 4 durch die Membran geführt, gewaschen und getrocknet.
100 µl eines 1 x Puffers für das Restriktionsenzym *Ava*I werden auf die Membran gegeben und
1) abgenommen, in ein neues Reaktionsgefäß überführt und anschließend ein Restriktionsenzym (beispielsweise *Ava*I der Fa. Promega) hinzugefügt;
2) ein Restriktionsenzym (beispielsweise *Ava*I der Fa. Promega) zum Eluat in der Säule hinzugefügt.

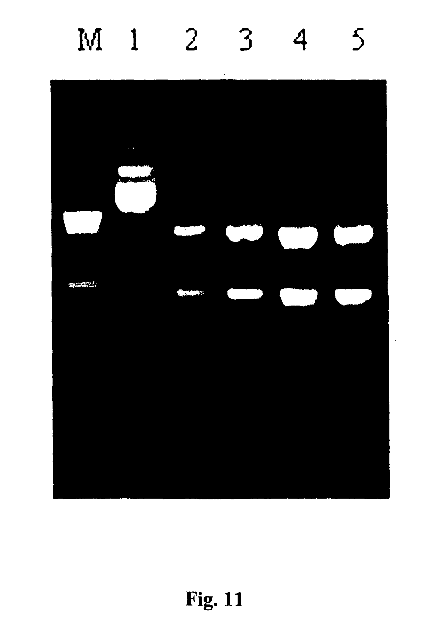

Die Reaktionen werden eine Stunde bei 37 °C inkubiert und anschließend auf ein nicht denaturierendes Gel aufgetragen und analysiert (Fig. 11).

Fig. 11 zeigt ein Ethidiumbromid-gefärbtes Agarcse-Gel einer elektrophoretischen Auftrennung des Plasmides pCMVβ nach Restriktion mit *Ava*I.
Spur 1: ungeschnittenes Plasmid;
Spur 2, 3: Elution mit dem Reaktionspuffer für *Ava*I, Restriktion in neuem Gefäß;
Spur 4, 5: Restriktion mit *Ava*I auf der Membran.

### Beispiel 2

### Druckfiltration zur Isopropanolfällung von DNA

Die Isolierung von Plasmid DNA wird nach Standardprotokollen bis einschließlich des Elutionsschritts über Anionenaustauscherchromatographie durchgeführt. Die DNA wird in einem Hochsalzpuffer von der Säule eluiert.

Anschließend werden zu dieser DNA-Lösung 0,7 Volumen Isopropanol zugegeben, der Ansatz gemischt und 1-5 Minuten bei Raumtemperatur inkubiert. Als Filtrationsvorrichtung wird ein 0,45 µm Celluloseacetat-Filter mit 5cm² Oberfläche in einer Filtrationskartusche (Standardvorrichtung zur Sterilfiltration, z.B. Minisart von Sartorius) verwendet. Dieser Filter wird auf eine Spritze gesteckt, bei der zunächst der Kolben entfernt wurde.

Das DNA-Isopropanolgemisch wird nun in die Spritze gefüllt und mit Hilfe des Kolbens durch den Filter gedrückt. Die DNA bleibt in dieser Präzipitat-Form zu einem hohen Prozentsatz auf dem Filter liegen (kann nicht durch die Poren treten).

Nun wird der Kolben wieder aus der Spritze entfernt, wieder eingefügt und Luft durch den Filter gedrückt.

Dieser Schritt wird 1-2 mal wiederholt und dient zur Trocknung der Membran.

Anschließend wird mit einem entsprechenden Volumen eines Niedrigsalzpuffers eluiert, indem der Puffer in den Spritzenkörper gefüllt und mit Hilfe des Kolbens durch den Filter gedrückt wird. Zur Erhöhung der Ausbeute wird dieses erste Eluat erneut in den Spritzenkörper gefüllt und mit Hilfe des Kolbens durch den Filter gedrückt.

Die erhaltenen Ausbeuten bewegen sich bei dieser Versuchsanordnung typischerweise im Bereich 80% bis 90% (siehe Beispiel 5).

### Beispiel 3

### Vakuumfiltration zur Isopropanolfällung von DNA

Wie bei der Druckfiltration wird hier zunächst Plasmid-DNA isoliert und mit 0,7 Vol Isopropanol versetzt. Als Filtrationsvorrichtung wird hier eine zur Vakuumfiltration konstruierte Apparatur verwendet, in die ein 0,45 µm Celluloseacetatfilter mit einer Oberfläche von 5 cm² eingespannt ist. Es können auch 0,45 µm Cellulosenitratfilter oder mehrere Lagen 0,65 µm Celluloseacetat oder Cellulosenitratfilter verwendet werden.

Das Isopropanol-DNA Gemisch wird 1-5 Minuten inkubiert und dann auf die Filtrationsvorrichtung gegeben. Durch Anlegen des Vakuums wird die Lösung durch den Filter gesaugt. Die DNA-Präzipitate auf dem Filter werden mit einem entsprechenden Volumen 70%igem Ethanol versetzt und durch erneutes Anlegen des Vakuums gewaschen. Die Elution der DNA vom Filter erfolgt durch Aufbringen eines Niedrigsalzpuffers, eine kurze Inkubation und erneutes Anlegen von Vakuum. Die Ausbeute kann entweder durch erneute Elution vom Filter mit einem zweiten Volumen Niedrigsalzpuffer oder durch erneute Elution mit dem Eluat aus dem ersten Elutionsschritt erfolgen.

Auch hier werden typischerweise Ausbeuten in der Größenordnung von 80% bis 90% der eingesetzten DNA erzielt.

### Beispiel 4

Als Methode wird die in Beispiel 3 angegebene Vakuumsfiltration benutzt. Als Filtrationsgefäß dient das Vakuumfiltrationsgerät Sartorius 16315. Als Plasmid DNA wird pCMVβ verwendet, welches aus DH5α isoliert worden war.

Jeweils 15 ml QF-Puffer (Hochsalzpuffer) werden mit 500 µg Plasmid versetzt und gemischt 10,5 ml Isopropanol werden zugegeben und es wird erneut gemischt. Dann wird für 5 Minuten inkubiert. Die so präzipitierte Plasmid-DNA wird auf den jeweils in die Filtrationsapparatur eingespannte Membran gegeben. Nun wird Vakuum angelegt und filtriert. Die Membranen werden mit 5 ml 70%igem Ethanol gewaschen (erneutes Anlegen von Vakuum). Sodann wird jeweils 1 ml TE-Puffer auf die Membranen pipettiert, 5 Minuten inkubiert und die DNA durch Anlegen von Vakuum eluiert. Anschließend erfolgt eine Nachelution mit 1 ml TE-Puffer. DNA-Gesamtmengen werden jeweils im Durchbruch, in der Waschfraktion und im vereinigten Eluat gemessen (OD260). Folgende Ergebnisse werden erhalten:

| Membran | Anzahl | Durchbruch | Waschfraktion | Eluat | Laufverhalten |
|---|---|---|---|---|---|
| PVDF 0.2 µm | 1 | 0 µg DNA | 0 µg DNA | 131 µg DNA | sehr langsam |
| | | | | | |
| Celluloseacetat | 3 | 0 µg DNA | 0 µg DNA | 469 µg DNA | schnell |
| 0.65 µm | | | | | |
| | | | | | |
| Celluosenitral | 2 | 0 µg DNA | 0 µg DNA | 418 µg DNA | schnell |
| 0.65 µm | | | | | |

Berechnet auf die 500 µg DNA-Ausgangsmenge ergeben sich in diesem Versuch folgende Ausbeuten:

| | |
|---|---|
| PVDF 0,2 µm | 26% |
| Celluloseacetat | 94% |
| 0.65 µm | |
| | |
| Cellulosenitrat | 84% |
| 0.65 µm | |

### Beispiel 5

Als Methode wird die in Beispiel 2 angegebene Druckfiltration benutzt. Als Filtrationsvorrichtung wird ein käuflicher 0,45 µm Celluloseacetatfilter (Minisart, Sartorius) verwendet. Als Plasmid DNA wird pCMVβ verwendet, welches aus DH5α isoliert worden war.

Jeweils 15 ml QF-Puffer (Hochsalzpuffer) werden mit 100, 200, 300, usw. bis 900 µg Plasmid versetzt und gemischt. 10,5 ml Isoproganol werden zugegeben und erneut gemischt. Anschließend erfolgt eine Inkubation für 5 Minuten. Die so präzipitierte Plasmid-DNA wird in eine Spritze überführt, an der vorher der Filter befestigt worden ist. Nun erfolgt Druckfiltration mit Hilfe der Spritze. Der Filter wird mit 2 ml 70%igem Ethanol gewaschen und 2x wie beschrieben getrocknet. Die Elution erfolgt mit 2 ml TE-Puffer. Es wird mit dem Eluat einmal nacheluiert. DNA-Gesamtmengen werden jeweils im vereinigten Eluat gemessen (OD260). Folgende Ergebnisse werden erhalten:

| Eingesetzte DNA-Menge | Eluierte DNA-Menge | %-Ausbeute |
|---|---|---|
| 100 µg | 100 µg | 100% |
| | | |
| 200 µg | 176 µg | 88% |
| | | |
| 300 µg | 257 µg | 86% |
| | | |
| 400 µg | 361 µg | 90% |
| 500 µg | 466 µg | 93% |
| | | |
| 600 µg | 579 µg | 97% |
| | | |
| 700 µg | 671 µg | 96% |
| | | |
| 800 µg | 705 µg | 88% |
| | | |
| 900 µg | 866 µg | 96% |

### Beispiel 6

Als Methode wird die in Beispiel 3 angegebene Vakuumfiltration benutzt. Als Fitrationsvorrichtung dient ein käuflicher 0,45 µm Celluloseacetatfilter (Minisart, Sartorius), der auf eine Filtrationskammer (QIAvac) gesteckt wird. Als Pufferreservoir wird ein Spritzenkörper am anderen Ende des Filters angebracht. Als Plasmid DNA wird pCMVβ verwendet, welches aus DH5α isoliert worden ist. 15 ml QF-Puffer (Hochsalzpuffer) werden mit 500 µg Plasmid versetzt und gemischt. 10,5 ml Isopropanol werden zugegeben und erneut gemischt. Anschließend erfolgt Inkubation für 5 Minuten. Die so präzipitierte Plasmid-DNA wird in den Spritzenkörper der Filtrationsapparatur gegeben. Nun wird Vakuum angelegt und filtriert. Der Filter wird nicht mit 70%igem Ethanol gewaschen. Vielmehr erfolgt unmittelbare Elution mit 2 ml Puffer EB (QIAGEN). Mit dem Eluat wird einmal nacheluiert. Die DNA-Gesamtmenge im vereinigten Eluat wird gemessen (OD260). Folgendes Ergebnis wird erhalten:

| Versuchsnummer | Eluierte DNA | % Ausbeute |
|---|---|---|
| 1 | 434 µg | 87% |
| | | |
| 2 | 437 µg | 87% |

## Patentansprüche

1. Verfahren zur Fällung von Plasmiden mit den folgenden Schritten:
- Bereitstellen eines Isoliergefäßes mit zumindest einer darin angeordneten Membran;
- Beschicken des Isoliergefäßes mit einer Plasmide enthaltenden Lösung;
- Präzipitation der in der Lösung enthaltenen Plasmide mit Alkohol, so dass sich die Plasmide an die zumindest eine Membran binden,
**dadurch gekennzeichnet, dass** die Porengröße der zumindest einen Membran größer oder gleich 0,45 Mikrometer beträgt und keine Zentrifugationsschritte durchgeführt werden, dass die Membran eine hydrophile oder hydrophilisierte Membran ist, und dass die Membran aus Celluloseacetat oder Cellulosenitrat besteht.

2. Verfahren nach Anspruch 1, **dadurch gekennzeichnet, dass** Alkohol vor dem Beschicken des Isoliergefäßes mit der Plasmide enthaltenden Lösung der Plasmide enthaltenden Lösung zugegeben wird.

3. Verfahren nach Anspruch 1, **dadurch gekennzeichnet, dass** Alkohol nach dem Beschicken des Isoliergefäßes mit der Plasmide enthaltenden Lösung der Plasmide enthaltenden Lösung zugegeben wird.

4. Verfahren nach einem der Ansprüche 1 bis 3, **dadurch gekennzeichnet, dass** die Fläche der Membran so gewählt ist, dass die gesamten in der Lösung enthaltenen Plasmide an die Membran binden können.

5. Verwendung von Membrane aus Celluloseacetat oder Cellulosenitrat mit einer Porengröße von größer oder gleich 0,45 Mikrometer zur Bindung von Alkohol-gefällten Plasmiden, wobei keine Zentrifugationsschritte durchgeführt werden.

6. Verfahren nach einem der Ansprüche 1 bis 4 oder Verwendung nach Anspruch 5, **dadurch gekennzeichnet, dass** als Alkohol vorzugsweise C1 - C5 Alkanole und besonders bevorzugt Isopropanol verwendet wird.

7. Verfahren nach einem der Ansprüche 1 bis 4 und 6 oder Verwendung nach Anspruch 5, **dadurch gekennzeichnet, dass** das Volumenverhältnis von Plasmid enthaltender Lösung zu Isopropanol 2:1 bis 1:1, vorzugsweise 1,67:1 bis 1:1, und besonders bevorzugt 1,43:1 bis 1:1 beträgt.

8. Verfahren oder Verwendung nach einem der Ansprüche 1 bis 7, **dadurch gekennzeichnet, dass** die Membran eine Porengröße von größer als 0,6 µm aufweist.

## Claims

1. A method for precipitating plasmids comprising the following steps:
- providing an insulating vessel with at least one membrane arranged therein;
- charging the insulating vessel with a solution containing plasmids;
- precipitation of the plasmids contained in the solution with alcohol so that the plasmids are bound to the at least one membrane,
**characterized in that** the pore size of the at least one membrane is greater than or equal to 0.45 micrometers and no centrifugation steps are performed, that the membrane is a hydrophilic or hydrophilized membrane, and that the membrane consists of cellulose acetate or cellulose nitrate.

2. The method according to claim 1, **characterized in that** alcohol is added to the solution containing plasmids before charging the insulating vessel with the solution containing plasmids.

3. The method according to claim 1, **characterized in that** alcohol is added to the solution containing plasmids after charging the insulating vessel with the solution containing plasmids.

4. The method according to one of the claims 1 to 3, **characterized in that** the surface of the membrane is selected such that all of the plasmids which are present in the solution can bind to the membrane.

5. Use of membranes made from cellulose acetate or cellulose nitrate and having a pore size of greater than or equal to 0.45 micrometers for binding alcohol precipitated plasmids, whereby no centrifugation steps are performed.

6. The method according to one of the claims 1 to 4 or the use according to claim 5, **characterized in that** preferably C1-C5-alkanols and particularly preferably isopropanol are used as alcohol.

7. The method according to one of the claims 1 to 4 and 6 or the use according to claim 5, **characterized in that** the volume ratio of plasmid containing solution to isopropanol is 2:1 to 1:1, preferably 1.67:1 to 1:1, and particularly preferably 1.43:1 to 1:1.

8. The method or use according to one of the claims 1 to 7, **characterized in that** the membrane has a pore size greater than 0.6 µm.

## Revendications

1. Procédé en vue de la précipitation de plasmides avec les étapes suivantes :
➢ mise à disposition d'un récipient d'isolation contenant au moins une membrane qui y est disposée ;
➢ chargement du récipient d'isolation avec une solution contenant les plasmides ;
➢ précipitation des plasmides contenus dans la solution à l'aide d'alcool, de manière à ce que les plasmides se lient à la au moins une membrane,
**caractérisé en ce que** la taille de pores de la au moins une membrane est supérieure ou égale à 0,45 micromètre et qu'aucune étape de centrifugation n'est effectuée, **en ce que** la membrane est une membrane hydrophile ou hydrophylisée, et **en ce que** la membrane se compose d'acétate de cellulose ou de nitrate de cellulose.

2. Procédé selon la revendication 1, **caractérisé en ce que** l'alcool est ajouté à la solution contenant les plasmides avant le chargement du récipient d'isolation avec la solution contenant les plasmides.

3. Procédé selon la revendication 1, **caractérisé en ce que** l'alcool est ajouté à la solution contenant les plasmides après le chargement du récipient d'isolation avec la solution contenant les plasmides.

4. Procédé selon l'une quelconque des revendications 1 à 3, **caractérisé en ce que** la surface de la membrane est choisie de telle sorte que la totalité des plasmides contenus dans la solution puissent se lier à la membrane.

5. Utilisation de membranes en acétate de cellulose ou nitrate de cellulose, avec une taille de pores supérieure ou égale à 0,45 micromètre pour lier les plasmides précipités à l'aide d'alcool, sans qu'aucune étape de centrifugation ne soit effectuée.

6. Procédé selon l'une quelconque des revendications 1 à 4 ou utilisation selon la revendication 5, **caractérisé en ce que** l'on utilise de préférence comme alcool les alcanols en C1-C5 et, de manière particulièrement préférée l'isopropanol.

7. Procédé selon l'une quelconque des revendications 1 à 4 et 6 ou utilisation selon la revendication 5, **caractérisé en ce que** le rapport volumique de la solution contenant les plasmides à l'isopropanol est de 2:1 à 1:1, de préférence, 1,67:1 à 1:1, et, de manière particulièrement préférée 1,43:1 à 1:1.

8. Procédé ou utilisation selon l'une quelconque des revendications 1 à 7, **caractérisé en ce que** la membrane a une taille de pores supérieure à 0,6 µm.
